# EUROPÄISCHE PATENTANMELDUNG

(11) **EP 1 025 855 A1**
(43) Veröffentlichungstag der Anmeldung: **09.08.2000**
(21) Anmeldenummer: 00102162.5
(22) Anmeldetag: 08.02.2000
(51) Int. Cl.: A61K 39/395, A61P 37/06

(54) **Arzneimittel enthaltend Anti-CD3 und Anti-Fcy-R-Antikörper zur begleitenden Behandlung bei Organtransplantationen**

(30) Priorität: 08.02.1999 DE 19905012
(71) Anmelder: GSF - Forschungszentrum für Umwelt und Gesundheit GmbH, 85764 Oberschleissheim (DE)
(72) Erfinder: Kummer, Udo, Dr., 80538 München (DE)
(74) Vertreter: Bösl, Raphael, Dr. rer. nat., Dipl.-Chem.

(57) **Zusammenfassung**

Die vorliegende Erfindung betrifft ein Arzneimittel enthaltend mindestens einen anti-T-Zellrezeptor/CD3-Antikörper und mindestens einen anti-Fcγ-Rezeptor-Antikörper zur Verringerung der Anzahl der T-Zellrezeptor/CD-3-Moleküle auf der Oberfläche von T-Zellen, insbesondere zur Immuntherapie, beispielsweise zur immunsuppressiven Behandlung bei Organtransplantationen, vor allem zur Unterdrückung oder Abschwächung der Zytokinkrankheit.

## Beschreibung

Die vorliegende Erfindung betrifft ein Arzneimittel enthaltend mindestens einen anti-T-Zellrezeptor/CD3-Antikörper und mindestens einen anti-Fcγ-Rezeptor-Antikörper zur Verringerung der Anzahl der T-Zellrezeptor/CD-3-Moleküle auf der Oberfläche von T-Zellen, insbesondere zur Immuntherapie, beispielsweise zur immunsuppressiven Behandlung bei Organtransplantationen, vor allem zur Unterdrückung oder Abschwächung der Zytokinkrankheit.

Zur Unterdrückung der Immunantwort im Rahmen einer Immuntherapie werden heutzutage hauptsächlich Kortikosteroide, Azathioprin, Zyklosporin A, Antithymozytenglobulin oder ein OKT3-Antikörper verwendet. Der monoklonale Antikörper OKT3 ist spezifisch für die ε-Kette des CD3-Komplexes, die mit dem T-Zell-Antigenrezeptor assoziiert ist. In der Immuntherapie wird OKT3 derzeit bei der Behandlung von Patienten nach Organtransplantation eingesetzt. Die Therapie mit dem OKT3 ist aber von unerwünschten und zum Teil fatalen Nebenwirkungen begleitet.

Ursache dieser Nebenwirkungen ist, daß OKT3-besetzte T-Zellen an entsprechende Rezeptoren auf Effektorzellen (Fcγ-Rezeptoren; FcγR) binden können und so die Interaktion des T-Zellrezeptors mit dem MHC-komplexierten Antigen nachahmen. Auf diese Weise werden die T-Zellen aktiviert und ihre Zellteilung in Gang gebracht. Dies führt zu einer Kettenreaktion, die eine massive Produktion und systemische Freisetzung von diversen Zytokinen aus der aktivierten T-Zelle veranlaßt. Die dann ausgeschütteten Zytokine, vornehmlich das Zytokin Interleukin-2, führen beim Menschen zu der gefürchteten Zytokinkrankheit, die den Symptomen des toxischen Schocks ähnelt und häufig intensivmedizinische Behandlung verlangt.

Diese Nebenwirkungen, die nur nach der Erstgabe des Antikörpers so vehement in Erscheinung treten, werden auch als 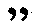 First-Dose-Syndrome" oder 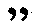 Cytokine-Release-Syndrome" bezeichnet, die den therapeutischen Einsatz von OKT3 erheblich einschränken.

Zur Vermeidung dieser toxischen Nebenwirkungen von OKT3 wurden daher Fc-freie Antikörperfragmente (anti-CD3-F(ab')₂ -Fragmente; siehe z.B. Blazar, B.R., et al. (1993) J. Immunol. 150, 265) hergestellt oder der Fc-Teil des Antikörpermoleküls mit Hilfe von molekularbiologischen Methoden, sog. Fc-Shuffling, so verändert, daß Interaktionen zwischen Antikörper-besetzten T-Zellen und den Fcγ-Rezeptoren auf den Effektorzellen entweder vollständig ausbleiben oder auf ein Minimum reduziert werden (siehe z.B. Alegre, M.-L. et al., (1995), J. Immunol. 155, 1544 oder Smith, J.-A. et al., (1997) J. Immunol. 185, 1413). Hierbei zeigt es sich, daß die so veränderten Antikörper über ein ähnlich großes immunsuppressives Potential verfügen, wie der intakte Antikörper oder im Falle des chimärischen Antikörpers sein parentales Pendant. Fc-freie bivalente-bindende F(ab')₂ -Antikörperfragmente werden üblicherweise durch Pepsineinwirkung unter sauren pH-Bedingung aus intakten IgG-Antikörpern hergestellt. Dies hat jedoch zur Folge, daß die so generierten Antikörperfragmente leicht denaturieren können. Zudem zeigen sich Unterschiede im Fragmentierungsverhalten der verschiedenen IgG-Subklassen. Daneben besitzen sowohl gentechnisch als auch durch Pepsinverdau hergestellte F(ab')₂ -Antikörperfragmente im Vergleich zu intakten IgG Antikörpern eine deutlich erhöhte Ausscheidungskinetik. Dies hat zur Folge, daß Einzeldosis und/oder Dosierungsintervalle entsprechend modifiziert werden müssen, damit der für den therapeutischen Erfolg erforderliche Blutspiegel des Immuntherapeutikums erreicht und auch längerfristig auf dem Niveau gehalten werden kann. Aus diesem Grund ist der breite klinische Einsatz der F(ab')₂ -Fragmente beschränkt.

Bei der Verwendung von durch Fc-Shuffling hergestellten nicht-mitogenen anti-CD3-Antikörpern hat sich im Mausmodell gezeigt, daß dieser chimärische anti-CD3-Antikörper nicht vollständig inert ist, was seine Interaktion mit den Fcγ-Rezeptoren betrifft. Die Interaktion mit den Fcγ-Rezeptoren führt zu einer partiellen Aktivierung der T-Zellen, wobei dieser Vorgang langfristig den funktionellen Status der Zellen verändert. Ein weiterer Nachteil aus der Interaktion mit den Fcγ-Rezeptoren besteht darin, daß das Ausmaß der Antikörperinduzierten Verringerung der Anzahl der T-Zellrezeptoren/CD3-Moleküle auf der Oberfläche von T-Zellen negativ beeinflußt und so das immunsupressive Potential des chimärischen Antikörpers eingeschränkt wird.

Zur Vermeidung der genannten toxischen Nebenwirkungen von OKT3 wurde auch ein Anti-T-Zellrezeptor-Antikörper der IgM-Klasse klinisch erprobt. Bei der Wahl dieser Antikörper-Klasse wird eine Interaktion mit Fcγ-Rezeptor-tragenden Effektorzellen vermieden. Erwartungsgemäß wurde der Antikörper von den Patienten besser vertragen als der murine IgG2a Antikörper OKT3. Jedoch ist es fraglich, ob der IgM-Antikörper noch über ein genügend großes immunsupressives Potential verfügt. Im Vergleich zu den bivalent bindenden IgG-Antikörpern verfügt ein IgM-Antikörper über maximal 5 x 2 Bindungsstellen. Ein Anti-TCR/CD3-Antikörper der IgM-Klasse bewirkt somit eine deutlich stärkere Vernetzung von T-Zellrezeptoren/CD3-Molekülen auf der Zelloberfläche, was, zumindest partiell, eine Aktivierung der T-Zelle induzieren könnte. Ein wesentliches Problem, das bei der Verwendung mit IgM-Antikörpern immer wieder auftritt besteht darin, daß diese Antikörper-Klasse durch die üblichen Aufreinigungsprozeduren aber auch durch Einfrieren, Auftauen oder allein durch die Lagerung in ihrer Aktivität deutlich geschwächt wird. Des Weiteren können Reinigungsprozeduren zur Denaturierung des Proteins führen, was mit einem Totalverlust der Aktivität des IgM-Antikörpers verbunden ist.

Aufgabe der vorliegenden Erfindung war daher, ein Mittel zur Immuntherapie bereitzustellen, das die oben beschriebenen Nachteile vermeidet bzw. verringert.

Ein wichtiger Mechanismus zur Ausschaltung immunkompetenter T-Zellen durch einen anti-T-Zellrezeptor/CD3-Antikörper besteht in der Verringerung der T-Zellrezeptor/CD3-Moleküle auf der Oberfläche der T-Zellen, wobei diese reaktionsunfähig werden. Hierbei gilt im allgemeinen, daß je mehr funktionelle T-Zellrezeptor/CD3-Moleküle von der Zelloberfläche verschwinden desto weniger ist die T-Zelle in der Lage, auf einen entsprechenden Stimulus zu reagieren. Überraschenderweise wurde nun gefunden, daß die Interaktion des Anti-T-Zellrezeptor/CD3-Antikörpers mit den Fcγ-Rezeptoren das Ausmaß der Verringerung der Anzahl der T-Zellrezeptor/CD3-Moleküle auf der Oberfläche von T-Zellen beeinflußt.

Ein Gegenstand der vorliegenden Erfindung ist daher (a) eine Zusammenstellung enthaltend mindestens einen anti-T-Zellrezeptor/CD3-Antikörper und mindestens einen anti-Fcγ-Rezeptor-Antikörper bzw. (b) eine Zusammenstellung enthaltend mindestens einen anti-T-Zellrezeptor/CD3-Antikörper und mindestens eine Mischung enthaltend mindestens einen Anti-T-Zellrezeptor/CD3 -Antikörper und mindestens einen anti-Fcγ-Rezeptor-Antikörper bzw. (c) eine Mischung enthaltend mindestens einen anti-T-Zellrezeptor/CD3 -Antikörper und mindestens einen anti-Fcγ-Rezeptor-Antikörper. Besonders bevorzugt sind anti-CD3 Antikörper der Bezeichnung 145-2C11 (DSM ACC 2378) und/oder KT3 (DSM ACC 2381) und der anti-Fcγ-Rezeptor Antikörper mit der Bezeichnung 2.4G2uk (DSM ACC 2379), hinterlegt in Form von Hybridomen bei der DSMZ- Deutsche Sammlung von Mikroorganismen und Zellkulturen GmbH, Mascheroder Weg 1b, D-38124 Braunschweig jeweils am 17.12.1998, oder Antikörper mit im wesentlichen gleichen oder ähnlichen Eigenschaften.

Erfindungsgemäß soll daher dem prospektiven Empfänger zusätzlich zu einem anti-TCR/CD3 -Antikörper ein zweiter Antikörper gleichzeitig oder zeitlich versetzt, insbesondere vorab, verabreicht werden, der gegen die Fcγ-Rezeptoren gerichtet ist. Hierbei sind vorzugsweise die beiden Antikörper aufeinander abgestimmt. So wird vorzugsweise die Subklasse des anti-T-Zellrezeptors/CD3-Antikörpers so ausgewählt, daß eine Interaktion ausschließlich mit den niedrig-affinen Fcγ-Rezeptoren, vor allem FcγRII und/oder FcγRIII, zustandekommt, während der anti-Fcγ-Rezeptor-Antikörper sich dadurch auszeichnet, daß er gegen die Ligandenbindungsstelle der niedrig-affinen Fcγ-Rezeptoren gerichtet ist, und so deren Funktion blockiert.

Durch die kombinierte Gabe von anti-T-Zellrezeptor-CD3-Antikörper und anti-Fcγ-Rezeptor-Antikörper wird das Andocken der Zell-gebundenen anti-T-Zellrezeptor/CD3-Antikörper an die Fcγ-Rezeptoren im wesentlichen unterbunden. Hierbei können die Konzentrationen der einzelnen Antikörper im wesentlichen gleich oder verschieden sein. Beispielsweise können die gleichen oder verschiedenen Antikörper in mindestens zwei verschiedenen Konzentrationen verwendet werden. Dieses Vorgehen gewährleistet daher zum einen, daß der Prozess der Antikörper-induzierten Verringerung von T-Zellrezeptor/CD3-Molekülen im wesentlichen nicht beeinträchtigt und mit der vorzugsweise maximalen Verringerung der T-Zellrezeptor/CD3-Moleküle eine entsprechende Reaktionsunfähigkeit bzw. Reaktionsverminderung der T-Zellen, insbesondere von CD4⁺ und CD8⁺ T-Zellen, erreicht wird. Zum anderen führt es dazu, daß die bei der heute gebräuchlichen anti-T-Zellrezeptor/CD3-Immuntherapie auftretenden Nebenwirkungen im wesentlichen vermieden werden können.

Ein weiterer Gegenstand der vorliegenden Erfindung ist daher ein Arzneimittel enthaltend eine erfindungsgemäße Zusammenstellung oder eine erfindungsgemäße Mischung sowie vorzugsweise geeignete Zusatz- und/oder Hilfsstoffe. Als Zusatz- und/oder Hilfsstoffe eignen sich beispielsweise eine physiologische Kochsalzlösung, geeignete Stabilisatoren, Proteinaseinhibitoren, etc. Als Stabilisatoren eignen sich beispielsweise Tween 80 (0,02 %), Zuckerlösungen, wie z. B. Saccharose-Lösung (20 - 30 %) oder Aminosäure-Lösungen, wie z. B. Glycin- oder Cystein-Lösungen. Üblicherweise wird das erfindungsgemäße Arzneimittel dadurch hergestellt, daß die erfindungsgemäße Zusammenstellung oder die erfindungsgemäße Mischung mit geeigneten Zusatz- und/oder Hilfsstoffen versetzt wird.

Ein weiterer Gegenstand der Erfindung ist auch die Verwendung der erfindungsgemäßen Zusammenstellung, der Mischung oder des Arzneimittels zur Verringerung der Anzahl der T-Zellrezeptor/CD3-Moleküle auf der Oberfläche von T-Zellen. Vorzugsweise wird das erfindungsgemäße Arzneimittel zur Immuntherapie, insbesondere von Autoimmunerkrankungen, sowie vorzugsweise zur immunsuppressiven Behandlung bei oder nach Organtransplantationen, vor allem mit dem Ziel zur Unterdrückung oder Abschwächung der oben näher beschriebenen Zytokinkrankheit verwendet.

Eine weitere bevorzugte Anwendung des erfindungsgemäßen Arzneimittels ist die Behandlung von T-Zellvermittelten Autoimmunerkrankungen, wie z. B. Erkrankungen des rheumatischen Formenkreises, juveniler Diabetes Mellitus (Typ I Diabetes), Multiple Sklerose, systemischer Lupus erythematodes, Polyarthritis nodosa, Wegener's Granulomatose, Sklerodermie, Morbus Reiter, Colitis ulcerosa und Morbus Crohn.

Um die Verringerung der T-Zellrezeptor/CD3-Moleküle auf der Oberfläche von T-Zellen im wesentlichen aufrechtzuerhalten, wird vorzugsweise der anti-T-Zellrezeptor/CD3 -Antikörper während der gesamten Therapiedauer kontinuierlich weitergegeben (Erhaltungsdosis), wohingegen das Bindungsverhalten zwischen dem anti-T-Zellrezeptor/CD3-Molekül und dem niedrig-affinen Fcγ-Rezeptoren, die nur im wesentlichen einmalige Gabe des anti-Fcγ-Rezeptor-Antikörpers zu Beginn der Immuntherapie erfordert (Induktionsphase). Im allgemeinen liegen die nach erfolgter Anfangsbehandlung verbliebenen Anti-T-Zellrezeptor/CD3-Antikörper in so geringer Kopienzahl auf der Zelloberfläche vor, daß eine Aggregation im wesentlichen nicht mehr zustandekommen kann und von daher die nachfolgende Interaktion mit den niedrig-affinen Fcγ-Rezeptoren ausbleibt. Aus diesem Grund kann die erfindungsgemäße Zusammenstellung mindestens einen anti-T-Zellrezeptor/CD3-Antikörper und mindestens einen anti-Fcγ-Rezeptor-Antikörper in getrennter Form enthalten oder eine Mischung beider Antikörper oder mindestens einen anti-T-Zellrezeptor/CD3-Antikörper in getrennter Form von mindestens einer Mischung enthaltend mindestens einen anti-T-Zellrezeptor/CD3-Antikörper und mindestens einen anti-Fcγ-Rezeptor-Antikörper. Durch die erfindungsgemäße Zusammenstellung oder Mischung läßt sich somit auf vorteilhafte Weise eine individuelle Therapie bewerkstelligen.

Die übliche Behandlungsdauer des Patienten beträgt ca. 1 bis 9 Tage, vorzugsweise ca. 7 bis 8 Tage. Die Dosis des jeweiligen Antikörpers beträgt im allgemeinen ca. 0,3 bis ca. 4 mg/kg/Tag. Beispielsweise ist die Dosierung für die Initialphase ca. 250 mg/Patient an anti-Fcγ-Rezeptor-Antikörper und ca. 150 mg/Patient an anti-T-Zellrezeptor/CD3-Antikörper und für die Erhaltungsphase ca. 25 mg/Patient an anti-T-Zellrezeptor/CD3-Antikörper, wobei es ausreicht, diese Dosis jeden 3. Tag zu verabreichen.

Die erfindungsgemäße Kombinationstherapie entspricht vom Prinzip her dem Ansatz mit den Fc-freien bivalent bindenden F(ab')₂-Antikörperfragmenten. Zum einen bedeutet dies, daß sich das immunsupressive Potential der anti-T-Zellrezeptor/CD3-Antikörper durch die erfindungsgemäße Kombinationstherapie nicht wesentlich von dem der F(ab')₂-Antikörperfragmenten unterscheidet. Zum anderen ist sichergestellt, daß es weder bei den F(ab')₂-Antikörperfragmenten noch bei der erfindungsgemäßen Kombinationstherapie im wesentlichen zu einer Interaktion mit den Fcγ-Rezeptoren kommt, was zur Folge hat, daß die oben beschriebenen, unerwünschten und gefährlichen Nebenwirkungen ( 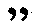 Cytokine-Release-Syndrome") im wesentlichen vollständig vermieden werden, was tierexperimentell nachgewiesen wurde. Zudem wurde in einem Kontrollexperiment nachgewiesen, daß die alleinige Gabe eines anti-Fcγ-Rezeptor-Antikörpers zu keinen erkennbaren Nebenwirkungen führte, vorzugsweise wenn durch die Wahl des Anti-Fcγ-Rezeptor-Antikörper-Isotyps gewährleistet wird, daß im wesentlichen keine Interaktion mit dem hoch-affinen Rezeptor für IgG (FcγRI) stattfinden kann.

Des Weiteren hat die erfindungsgemäße Kominationstherapie folgende Vorteile:
1. Die intakten Antikörper sind aufgrund ihrer günstigen pharmakokinetischen Eigenschaften (Halbwertzeit) den F(ab')₂-Antikörperfragmenten deutlich überlegen.
2. Bei der erfindunsgemäßen Kombinationstherapie kommt eine Interaktion des anti-T-Zellrezeptors/CD3-Antikörpers mit Fcγ-Rezeptoren im wesentlichen nicht zustande. Neben der Verringerung bzw. Ausschaltung unerwünschter Nebenwirkungen ist zudem mit einer neutralisierenden anti-Antikörper-Antwort gegen den T-Zellrezeptor/CD3-Antikörper nicht oder nur in abgeschwächter Form zu rechnen.
3. Die erfindungsgemäße Kombinationstherapie kann schnell und ohne unangenehme Langzeiteffekte beendet werden. So wurde gezeigt, daß anti-T-Zellrezeptor/CD3-Antikörper, die in Kombination mit einem anti-Fcγ-Rezeptor-Antikörper verabreicht wurden, zu einer signifikanten Verringerung von T-Zellrezeptor-CD3-Molekülen führte, ohne den funktionellen Status der T-Zelle im wesentlichen zu verändern. Des Weiteren wurde festgestellt, daß nach erneuter Expression der T-Zellrezeptor/CD3-Moleküle auf der Zelloberfläche wenige Stunden nach Absetzen der erfindungsgemäßen Immuntherapie die immunologische Reaktionsfähigkeit der T-Zelle sofort und vollständig wiederhergestellt war.
4. Ein weiterer Vorteil der erfindungsgemäßen Kominationstherapie ist, daß der Anti-Fcγ-Rezeptor-Antikörper nur während der Induktionsphase, d. h. zu Beginn der Therapie, verabreicht zu werden braucht. In der sich anschließenden Erhaltungsphase ist es ausreichend, nur noch den anti-T-Zellrezeptor/CD3-Antikörper zu injizieren, wobei vorteilhafterweise eine so geringe Dosierung gewählt werden kann, daß gerade noch eine Verringerung der Anzahl der T-Zellrezeptor/CD3-Moleküle auf der Oberfläche von T-Zellen gewährleistet ist.

Die folgenden Figuren und Beispiele sollen die Erfindung näher erläutern ohne sie zu beschränken.

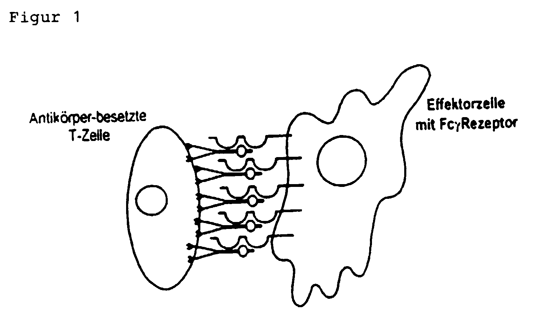

- Fig. 1: beschreibt schematisch eine übliche Immuntherapie mit dem Antikörper OKT3. Hierbei führt die Konjugatformation zwischen Antikörper-besetzter T-Zellen und Fcγ-Rezeptor-tragender Effektorzelle zum sogenannten 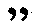 Cytokine-Release-Syndrome".

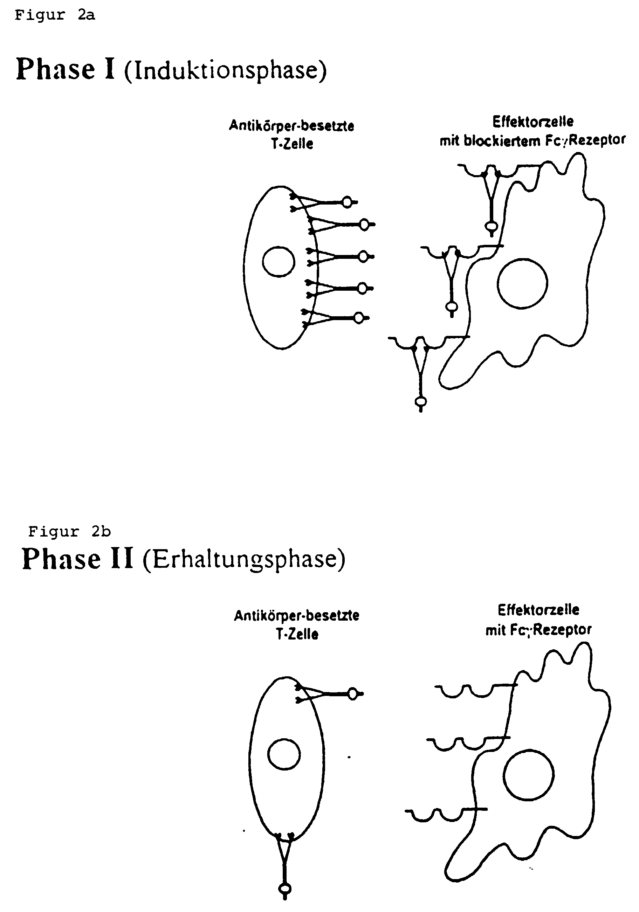

- Fig. 2: beschreibt schematisch die erfindungsgemäße Immuntherapie durch eine kombinierte Gabe von Antikörpern gegen T-Zellrezeptor/CD3-Komplex und niedrig-affine Fcγ-Rezeptoren.
- Fig.2a: zeigt hierbei die Phase I (Induktionsphase), bei der eine Verringerung von T-Zellrezeptor/CD3 -Molekülen durch anti-T-Zellrezeptor/CD3 -Antikörper erfolgt. Eine Interaktion mit der Effektorzelle und somit das 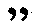 Cytokine-Release-Syndrome" wird vermieden.
- Fig.2b: zeigt die Phase II (Erhaltungsphase), bei der die Erhaltungsdosis von anti-T-Zellrezeptor/CD3-Antikörper zur Aufrechterhaltung der Verringerung der Anzahl der T-Zellrezeptor/CD3-Moleküle auf der Oberfläche von T-Zellen eine Reaktionsunfähigkeit der T-Zellen bewirkt. Eine Interaktion mit der Effektorzelle findet nicht statt.

### BEISPIELE

### 1. Materialien

Für alle in vivo Experimente wurden CBA/J-Mäuse (The Jackson Laboratory, Animal Resources, 600 Main Street, Bar Harbor, Main, 04609-1500, USA) verwendet, und unter spezifisch pathogen-freien Bedingungen gehalten. Für die Untersuchungen wurden weibliche Tiere im Alter von 3 Monaten verwendet.

Die Antikörper wurden aus dem Kulturüberstand von Hybridomzellinien entweder durch konventionelle Protein G (17A2, KT3) oder Protein A (7D6, 145-2C11) Affinitätschromatographie (Pharmacia, Uppsala, Schweden) gereinigt. Die biologische Aktivität jeder Probe wurde vorab im Fluoreszenz-aktivierten Zellanalysator (FACS, Becton Dickinson, Heidelberg, Deutschland) getestet.

### 2. Behandlungsprotokoll

Die anti-TCR CD3-Antikörper 7D6(DSM ACC2382) , 17A2(DSM ACC2380), 145-2C11 und KT3 (400 µg anti-TCR/CD3-Antikörper pro Tier in 0,5 ml Phosphat-gepufferter Kochsalzlösung; PBS) oder Antikörper-Cocktails bestehend aus den einzelnen anti-TCR/CD3-Antikörpern, zusammen mit dem anti-FcγR-Antikörper 2.4G2uk (400 µg anti-TCR/CD3-Antikörper + 1,0 mg 2.4G2 pro Tier in 0,5 ml PBS) wurde den Versuchsmäusen als einmalige intraperitoneale Injektion verabreicht. Zu den angegebenen Zeitpunkten wurden die Milz der so behandelten Tiere gewonnen.

### 3. Anfärben der Zellen

Für den Nachweis von spezifischen Oberflächen-Antigenen auf den Milzzellen der Versuchstiere wurde zunächst eine Einzelsuspension hergestellt. Anschließend wurden die Erythrozyten lysiert und die verbliebenen Zellen in PBS mit 2 %igem Hitze-inaktivierten fötalem Kälberserum (FCS) und 0,1 %igem Natriumazid (NaN₃) aufgenommen. Das Anfärben der Zellen erfolgte nach einem Standardprotokoll. Dazu wurden 10⁶ Zellen in 50 µl Waschpuffer (PBS/2 % FCS/0,1 % NaN₃) mit 50 µl (sättigende Konzentrationen an Nachweis-Antikörpern) des Fluoreszenz-markierten anti-TCR-Antikörpers H57-597 (DSM ACC2384) entweder zusammen mit einem Phykoerythrin-konjugierten anti-CD4-Antikörper (Becton Dickinson) oder einem Phykoerythrin-konjugierten anti-CD8-Antikörper (Medac, Hamburg, Deutschland) zusammengegeben. Nach einer Inkubation von 30 Minuten und einem Wasch-Zentrifugationsschritt wurden die Zellen in 300 µl Waschpuffer aufgenommen und mit Hilfe eines FACS-Gerätes (Becton Dickinson) analysiert. Zum Ausschluß von toten Zellen wurde jedem Ansatz Propidiumjodid (30 µl) zugegeben. Bei jedem Probenlauf wurden mindestens 10⁴ Leukozyten berücksichtigt und die Ergebnisse mit Hilfe der Software Lysis II (Becton Dickinson) ausgewertet.

## Patentansprüche

1. Zusammenstellung enthaltend mindestens einen anti-T-Zellrezeptor/CD3-Antikörper und mindestens einen anti-Fcγ-Rezeptor-Antikörper.

2. Zusammenstellung enthaltend mindestens einen anti-T-Zellrezeptor/CD3 -Antikörper und mindestens eine Mischung enthaltend mindestens einen anti-T-Zellrezeptor/CD3-Antikörper und mindestens einen anti-Fcγ-Rezeptor-Antikörper.

3. Mischung enthaltend mindestens einen anti-T-Zellrezeptor/CD3-Antikörper und mindestens einen anti-Fcγ-Rezeptor-Antikörper.

4. Zusammenstellung nach Anspruch 1 oder 2 und/oder Mischung nach Anspruch 3, dadurch gekennzeichnet, daß der anti-T-Zellrezeptor/CD3-Antikörper mit niedrig-affinen Fcγ-Rezeptoren, vorzugsweise mit Fcγ-RII und/oder Fcγ-RIII, interagiert.

5. Zusammenstellung oder Mischung nach einem der Ansprüche 1-4, dadurch gekennzeichnet, daß der anti-Fcγ-Rezeptor-Antikörper mit niedrig-affinen Fcγ-Rezeptoren, vorzugsweise mit Fcγ-RII und/oder Fcγ-RIII, interagiert.

6. Zusammenstellung oder Mischung nach einem der Ansprüche 1-5, dadurch gekennzeichnet, daß der anti-T-Zellrezeptor/CD3-Antikörper und/oder der anti-Fcγ-Rezeptor-Antikörper in mindestens zwei verschiedenen Konzentrationen enthalten ist.

7. Arzneimittel enthaltend eine Zusammenstellung oder eine Mischung gemäß einem der Ansprüche 1-6 sowie vorzugsweise geeignete Zusatz- und/oder Hilfsstoffe.

8. Verfahren zur Herstellung eines Arzneimittels gemäß Anspruch 7, dadurch gekennzeichnet, daß eine Zusammenstellung oder eine Mischung gemäß einem der Ansprüche 1-6 mit geeigneten Zusatz- und/oder Hilfsstoffen versetzt wird.

9. Verwendung einer Zusammenstellung, einer Mischung oder eines Arzneimittels gemäß mindestens einem der Ansprüche 1-7 zur Verringerung der Anzahl der T-Zellrezeptor/CD3-Moleküle auf der Oberfläche von T-Zellen.

10. Verwendung eines Arzneimittels gemäß Anspruch 7 zur Immuntherapie, insbesondere von Autoimmunerkrankungen, vor allem von T-Zellvermittelten Autoimmunerkrankungen.

11. Verwendung eines Arzneimittels gemäß Anspruch 7 oder 10 zur immunsuppressiven Behandlung bei und/oder nach Organtransplantationen.

12. Verwendung eines Arzneimittels gemäß mindestens einem der Ansprüche 7, 9, 10 oder 11 zur Unterdrückung oder Abschwächung der Zytokinkrankheit.

13. Verwendung eines Arzneimittels gemäß Anspruch 10 zur Behandlung von T-Zellvermittelten Autoimmunerkrankungen, insbesondere Erkrankungen des rheumatischen Formenkreises, juveniler Diabetes Mellitus (Typ I Diabetes), Multiple Sklerose, systemischer Lupus erythematodes, Polyarthritis nodosa, Wegeners Granulomatose, Sklerodermie, Morbus Reiter, Colitis ulcerosa und Morbus Crohn.
